# EUROPÄISCHE PATENTSCHRIFT

(11) **EP 0 593 974 B1**
(45) Veröffentlichungstag und Bekanntmachung des Hinweises auf die Patenterteilung: **19.06.1996**
(21) Anmeldenummer: 93116143.4
(22) Anmeldetag: 06.10.1993
(51) Int. Cl.: C07C 327/22, C08F 20/38

(54) **Monomere für hochlichtbrechende Kunststoffe**
Monomers for high refractive index plastics
Monomères pour des matières plastiques

(30) Priorität: 10.10.1992 DE 4234253
(43) Veröffentlichungstag der Anmeldung: 27.04.1994
(73) Patentinhaber: RÖHM GMBH, D-64293 Darmstadt (DE)
(72) Erfinder: Bader, Martina, Dr., D-64347 Griesheim (DE); Kerscher, Volker, Dr., D-64354 Reinheim (DE)

(56) Entgegenhaltungen:
- EP-A- 0 273 661
- PATENT ABSTRACTS OF JAPAN vol. 12, no. 438 (C-544)17. November 1988 & JP-A-63 162 671 (NIPPON SHOKUBAI KAGAKU KOGYO)
- PATENT ABSTRACTS OF JAPAN vol. 13, no. 331 (C-622)25. Juli 1989 & JP-A-01 110 666 (NIPPON SHOKUBAI KAGAKU KOGYO)
- PATENT ABSTRACTS OF JAPAN vol. 14, no. 439 (C-761)19. September 1990 & JP-A-02 172 969 (MITSUI TOATSU CHEMICALS)

## Beschreibung

### Gebiet der Erfindung

Die Erfindung betrifft Monomere für hochlichtbrechende Kunststoffe aus der Gruppe der Dithio(meth)acrylsäureester.

### Stand der Technik

Schwefelhaltige Monomere bieten sich an zur Herstellung hochlichtbrechenden optischen Materials, da der Schwefel aufgrund seiner leichten Polarisierbarkeit eine starke Wechselwirkung der Materie mit eingestrahltem Licht bewirkt, was sich in einer hohen Brechzahl widerspiegelt.
Aliphatische Thioether(meth)acrylate, auch bifunktionelle, wie sie z.B. den Gegenstand der DE-A 38 38 350 bilden, haben einen nach oben limitierten Schwefelgehalt (laut Beispielen maximal 27 Gew.-% Schwefel für acetalfreie Strukturen) und erlauben somit für ein Polymerisat lediglich einen Brechungsindex < 1,58.
Aromatische Strukturelemente tragen zwar ebenfalls zur Erhöhung der Brechzahl bei, aber auch zur Erhöhung der Dispersion. Angestrebt wird aber eine geringe Dispersion, um die chromatische Aberration des Polymerisats möglichst klein zu halten.
Einen höheren Schwefelgehalt, auch in relativ einfachen Strukturen kann man mit Estern der Thio(meth)acrylsäure erzielen. So weisen die in EP-A 273 661 benannten Alkandithiomethacrylate einen Schwefelgehalt von bis zu 39 Gew.-% auf. Die Ethylsulfid-Partialstruktur (Thioetherthiomethacrylate) bringt jedoch bei der Herstellung Probleme mit sich: Die den Dimethacrylaten zugrundeliegenden Dimercaptane lassen sich nur in unbefriedigenden Ausbeuten herstellen und reinigen, da sich dabei leicht - z.T. unter Zersetzung des höheren Dimercaptans - das 1,4-Dithian bildet. Eine Reinigung des Mercaptans als Ausgangsprodukt erscheint jedoch unumgänglich, da die herzustellenden Dithio(meth)acrylate im technischen Prozeß aufgrund des hohen Siedepunkts kaum noch zu reinigen sind, d.h. auf der Stufe der Endprodukte keine Abtrennung des Neben- bzw. Abbauproduktes mehr möglich ist.

### Aufgabe und Lösung

Es bestand somit weiterhin ein Bedarf an Monomeren mit einem hohen Schwefelgehalt, die leicht zugänglich, farblos und bei Raumtemperatur flüssig sein sollten.

Es wurde nun gefunden, daß durch Einbau von Alkylidenbrücken mit mehr als zwei Kohlenstoffatomen zwischen den Schwefelatomen rein herstellbare Dimercaptane zugänglich sind, die sich zu den entsprechenden Dithio(meth)acrylaten umsetzen lassen.

Die vorliegende Erfindung betrifft somit Dithio(meth)acrylsäureester der allgemeinen Formel I 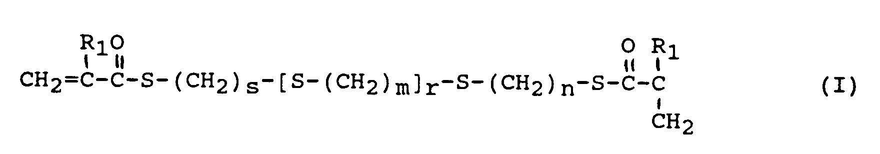
worin
- R₁: für Wasserstoff oder Methyl und
- r: für O oder 1 steht und s, m und n für eine ganze Zahl von 2 bis 6 stehen mit der Maßgabe, daß für r gleich 0 die Summe aus s und n größer 4 und für r gleich 1 die Summe aus s, m und n größer 6 sein soll.

Die Abkehr von der Ethylbrücke als alleiniger repetierender Einheit bewirkt, daß sich weniger Nebenprodukte infolge Cyclisierung bilden, weil die Bildungstendenz für mittlere Ringe (7-9-Ringe) weitaus geringer ist als für den Sechsring des Dithians. Es hat sich zudem erwiesen, daß flüssige Dimercaptane als Edukte günstiger sind für den Herstellungsprozeß: Bei der Einführung der Thiogruppe mittels Thioharnstoff kommt es bei festen Dimercaptanen zu Abtrennungsproblemen und somit zu einem erhöhten Lösemittelbedarf. So ist das 1,2-Bis(2-mercaptoethylthio)ethan ein Feststoff, während das 1,3-Bis(2-mercaptoethylthio)propan als Flüssigkeit anfällt.
Besonders genannt seien als Dithio(meth)acrylsäureester das 1,3-Bis(2-methacrylthioethylthio)propan (Vbdg. der Formel I mit r = 1, m = 3, s,n = 2) und 1,6-Bis(methacrylthio)-3-thiahexan (Vbdg. der Formel I mit r = 0, S = 3, n = 2).

Die vorliegende Erfindung betrifft ferner ein Verfahren zur Herstellung der Verbindungen der allgemeinen Formel I, wobei man das Dithiol der allgemeinen Formel II

MS-(CH₂)ₛ-[S-(CH₂)ₘ]ᵣ-S-(CH₂)ₙ-SM (II)

worin r, s, m und n die oben bezeichneten Bedeutungen besitzen und M für Wasserstoff oder ein
Metallkation, vorzugsweise ein Alkalikation steht, mit mindestens der doppelt molaren Menge eines (Meth)acrylsäurederivats der Formel III 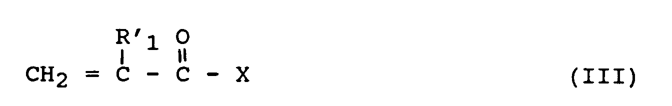
worin
- X: für 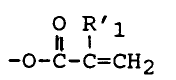 oder für Cl steht und
- R'₁: Wasserstoff oder Methyl bedeutet,
umsetzt.
Zweckmäßigerweise setzt man das (Meth)acrylsäureanhydrid bzw. das -chlorid in einem geeigneten, inerten, vorzugsweise nicht mit Wasser mischbaren Lösungsmittel L, wie z.B. einem Ether wie Methyl-tert.butylether oder einem aromatischen Lösemittel wie Toluol, Xylol, um.
Vorzugsweise wendet man das Dithiol der Formel II in wäßrig-alkalischer Lösung an, so daß bei der Umsetzung gebildete Säure HX (wenn M = H) neutralisiert wird.

Die Dithiole der allgemeinen Formel (II) lassen sich in an sich bekannter Weise gewinnen. Das Verfahren sei am Beispiel des 3,7-Dithianonan-1,9-dithiols näher erläutert.
Zunächst löst man 2-Mercaptoethanol in ethanolischer Natronlauge vorteilhafterweise unter einem inerten Schutzgas wie Stickstoff und unter Eiskühlung, und tropft dazu unter Rühren bei ca. 50 Grad C die halbe molare Menge an 1,3-Dibrompropan zu. Nach einigen Stunden Reaktionszeit, als Anhalt seien etwa 6 Stunden genannt, wird vom entstandenen Salz abfiltriert, die Rohlösung mit einem inerten, nicht mit Wasser mischbaren Lösungsmittel wie etwa Methylenchlorid versetzt und erneut filtriert.
Die weitere Aufarbeitung des gebildeten 3,7-Dithianonan-1,9-diols geschieht vorzugsweise durch fraktionierte Destillation im Hochvakuum. Das so erhaltene Diol (1 Mol) wird zusammen mit ca. 2 mol Thioharnstoff in konzentrierter Salzsäure und unter Stickstoff ca. 8 Stunden am Rückfluß erhitzt. Sodann wird unter Eiskühlung Alkalilauge, vorzugsweise Kalilauge zugegeben und erneut - etwa 3 Stunden - unter Rückfluß erhitzt. Aus dem abgekühlten Reaktionsgemisch wird die organische Phase abgetrennt und die wäßrige Phase nach Ansäuern mit einem inerten nicht-wasserlöslichen Lösemittel wie z.B. Methyltert.butylether extrahiert. Die organischen Phasen werden vereinigt. Nach dem Trocknen und Verdampfen des Lösemittels wird das Dithiol durch Hochvakuumdestillation als farblose Flüssigkeit gewonnen.

### Herstellung der Dithio(meth)acrylsäureester der Formel (I)

Die Herstellung der Ausgangsverbindungen der Formel (III) ist seit langem bekannt (vgl. H. Rauch-Puntigam, Th. Völker, Acryl- und Methacrylverbindungen, Springer-Verlag, Heidelberg, New York 1967). Vorzugsweise setzt man die Verbindung der Formel III, insbesondere das (Meth)acrylsäureanhydrid in einem gewissen molaren Überschuß, beispielsweise im 0,05 bis 0,5-fachen Überschuß über die stöchiometrisch erforderliche Menge ein, zweckmäßigerweise stabilisiert mit einem an sich bekannten Polymerisationsinhibitor, beispielsweise einem sterisch gehinderten Phenol wie dem 4-Methyl-2,6-ditert.butylphenol (vgl. R. Gaechter u. H. Müller, Taschenbuch der Kunststoff-Additive, Hanser-Verlag 1979).

Bei der Umsetzung geht man zweckmäßig so vor, daß man die unter Eiskühlung hergestellte Lösung der Verbindung der Formel II in wäßrigem Alkali, beispielsweise in 10 %-iger Natronlauge (etwa 0,1 mol auf 100 ml) unter Rühren zu einer Lösung der Verbindung der Formel III, vorzugsweise des (Meth)acrylsäureanhydrids in dem inerten Lösungsmittel L beispielsweise in Methyl-tert.butylether (etwa 1 g auf 6-7 ml Lösemittel) zweckmäßig bei 15 - 20 Grad C zutropft.
Man rührt noch einige Stunden bei erhöhter Temperatur nach, beispielsweise 3 Stunden bei 45 Grad C. Nach Abtrennung der organischen Phase und zweckmäßig Waschen derselben mit Wasser wird mit einem geeigneten Trockenmittel, beispielsweise Natriumsulfat getrocknet und das Lösemittel durch Verdampfen entfernt.
Man erhält so unmittelbar die Verbindung der Formel I.

### Vorteilhafte Eigenschaften

Die erfindungsgemäßen Monomeren der allgemeinen Formel (I) lassen sich nach dem Verfahren der radikalischen Polymerisation (s. H. Rauch-Puntigam, Th. Völker, loc.cit.) z.B. zu transparenten, farblosen oder im Bedarfsfalle eingefärbten hochlichtbrechenden Kunststoffen polymerisieren.
Derartige Kunststoffe sind für eine Vielfalt optischer Gerätschaften und Artikel geeignet, beispielsweise für Linsen, Prismen, Brillengläser u.ä.

Die folgenden Beispiele dienen zur Erläuterung der Erfindung.

### BEISPIELE

### Beispiel 1: Herstellung von 1,3-Bis(2-methacrylthioethylthio)propan (Formel I; mit R₁ = CH, r = 1; m = 3; s,n = 2)

Eine unter Eiskühlung hergestellte Lösung von 0,44 mol 3,7-Dithianonan-1,9-dithiol in 400 ml 10 %-iger Natronlauge wird bei 15 - 20 Grad C und unter Rühren zu einer Lösung von 150 g Methacrylsäureanhydrid in 950 ml Methyl-tert.butylether in Gegenwart von 3000 ppm 4-Methyl-2,6-ditert.butylphenol zugetropft. Man rührt noch 3 Stunden bei 45 Grad C nach, trennt die organische Phase im Scheidetrichter ab und wäscht mit Wasser. Nach dem Trocknen mit Natriumsulfat und Entfernung des Lösemittels auf dem Rotationsverdampfer erhält man 150 g an dem Methacrylsäureester der Formel I (94 % d.Th.).

### Beispiel 2: Herstellung des 3,7-Dithianonan-1,9-dithiols

### (a) Herstellung des 3,7-Dithianonan-1,9-diols

Unter Eiskühlung und unter Stickstoffatmosphäre werden 312 g (4 mol) 2-Mercaptoethanol in ethanolischer Natronlauge (161 g in 2,2l Ethanol) vorgelegt. Zu dieser Lösung werden bei 50 Grad C 2 mol Dibrompropan zugetropft. Nach 6 Stunden Reaktionszeit wird vom gebildeten Salz abfiltriert, die Rohlösung mit 600 ml Methylenchlorid versetzt und erneut filtriert. Nach fraktionierter Destillation erhält man 328 g des 2,7-Dithianonan-1,9-diols (84 % d.Th., Kp. 167 - 169 Grad C/2mbar. Reinheit aufgrund von gaschromatographisch-massenspektroskopischer Prüfung > 95 %).

### (b) Herstellung des 3,7-Dithianonan-1,9-dithiols

140,5 g (0,72 mol) des Diols aus (a), 120 g Thioharnstoff sowie 376 g konz. Salzsäure werden unter Stickstoff 8 Stunden am Wasserbad zum Rückfluß erhitzt. Unter Eiskühlung wird dann Kalilauge (268 g KOH auf 1,7 l Wasser) zugegeben und 3 Stunden unter Rückfluß erhitzt.
Das Reaktionsgemisch wird nach dem Abkühlen im Scheidetrichter getrennt und die wäßrige Phase wird nach Ansäuern mit verd. Salzsäure mit Methyl-tert.butylether extrahiert. Die vereinigten organischen Phasen werden nach der Trocknung mit Natriumsulfat destilliert.
Man erhält 136 g (73 % d.Th) des farblosen Dithiols (Kp. 140 - 150 Grad C/0,1 mbar).

### Beispiel 3: Herstellung des 3-Thiahexan-1,6-dithiols

### a) Herstellung des 3-Thiahexan-1,6-diols

Unter Rühren und Stickstoffeinleitung werden 390 g (5 mol) 2-Mercaptoethanol mit 0,68 g Azobis(isobutyronitril) versetzt und eine Stunde lang auf 60 Grad C erwärmt. Unter Einhaltung dieser Temperatur werden innerhalb von 4 Stunden 290 g (5 mol) Allylalkohol zugetropft. Nach Zutropfende und Abklingen der exothermen Reaktion läßt man noch 1,5 Stunden bei 75 Grad C nachreagieren und destilliert das Rohprodukt im Vakuum (Kp: 118 - 121 Grad C, 0,2 - 0,4 mbar). Man erhält 588 g des 3-Thiahexan-1,6-diols als leicht gelb gefärbte Flüssigkeit (86 % d.Th., Reinheit nach gaschromatrographischer Prüfung > 95 %).

### b) Herstellung des 3-Thiahexan-1,6-dithiols

Unter Rühren und Stickstoffeinleitung wird ein Gemisch aus 136 g (1 mol) 3-Thiahexan-1,6-diol aus (a), 168 g (2,2 mol) Thioharnstoff und 0,525 l (5,4 mol) konz. Salzsäure 16 Stunden unter Rückfluß erhitzt. Nach dem Abkühlen tropft man unter Kühlung bei ca. 20 Grad C innerhalb von 1 - 1,5 Stunden eine Lösung von 333 g (5,9 mol) Kaliumhydroxid in 1,9 l Wasser zu und erhitzt anschließend 3 Stunden zum Rückfluß. Das abgekühlte Reaktionsgemisch wird mit Methyl-tert.butylether extrahiert. Nach Entfernen des Extraktionsmittels am Rotationsverdampfer wird das Rohmercaptan fraktioniert destilliert (Kp: 92 - 96 Grad C, 0,1 - 0,3 mbar).
Man erhält 92 g des farblosen 3-Thiahexan-1,6-dithiols (55 % d.Th., Reinheit nach gaschromatographischer Prüfung > 97 %. )

### Beispiel 4 Herstellung von 1,6-Bis(methacrylthioethyl)-3-thiahexan

Unter Lufteinleitung, Rühren und Kühlung wird bei 15 - 20 Grad C zu einer Lösung von 500 g (3,3 mol) Methacrylsäureanhydrid und 500 mg 2,5-Ditert.butyl-4-methylphenol in 1,6 l Methyl-tert.butylether eine Lösung von 252 g (1,5 mol) 3-Thiahexan-1,6-dithiol und 144 g (3,6 mol) Natriumhydroxid in 1,4 l Wasser zugetropft.
Anschließend läßt man 3 Stunden bei 40 Grad C nachreagieren. Nach dem Abkühlen wird die organische Phase im Scheidetrichter abgetrennt und mit 3 x 0,6 l Wasser gewaschen. Nach dem Trocknen mit Natriumsulfat und dem Entfernen des Lösemittels am Rotationsverdampfer unter Zusatz von 750 ppm 2,5-Ditert.butyl-4-methylphenol erhält man 415 g (92 % d.Th. ) 1,6-Bis(methacrylthio)-3-thiahexan als ölige Flüssigkeit.

### Beispiel 5 Polymerisat aus 1,3-Bis(2-methacrylthioethylthio)propan

32 g 1,3-Bis(2-methacrylthioethylthio)propan, welches gemäß Beispiel 1 hergestellt wurde, wird mit 118 mg Azobis(isobutyronitril) versetzt. Das Gemisch wird zwischen zwei Glasplatten (ca 90 x 120 x 3 mm) verfüllt und innerhalb 28 Stunden in einem Wasserbad mit einem Temperaturverlauf von 40 - 90 Grad C polymerisiert. Die klare, harte Kunststoffplatte hat eine Brechzahl von 1,6219 und eine Abbezahl von 38,6.

### Beispiel 6 Copolymerisat aus Methylmethacrylat und 1,6-Bis(methacrylthio)-3-thiahexan

Die Monomermischung aus 28 g 1,6-Bis(methacrylthio)-3-thiahexan und 3,1 g Methylmethacrylat wird mit 63 mg Azobis(isobutyronitril) versetzt und wie oben polymerisiert. Die klare, harte Kunststoffplatte hat eine Brechzahl von 1,5998 und eine Abbezahl von 41,1.

## Patentansprüche

1. Dithio(meth)acrylsäureester der allgemeinen Formel (I) 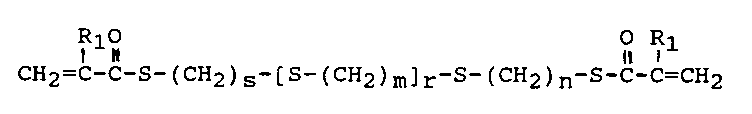 worin
R₁ für Wasserstoff oder Methyl und
r für null oder 1 steht und s, m und n für eine ganze Zahl von 2 bis 6 stehen mit der Maßgabe, daß sofern r für null steht, die Summe aus s und n größer 4 und sofern r für 1 steht, die Summe aus s, m und n größer 6 sein soll.

2. 1,3-Bis(2-methacrylthioethylthio)propan

3. 1,6-Bis(methacrylthio)-3-thiahexan

4. Verfahren zur Herstellung von Dithio(meth)acrylsäureestern der allgemeinen Formel I 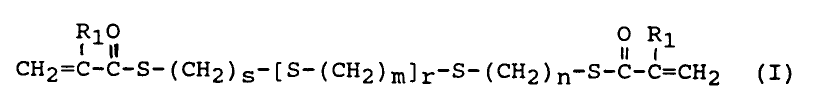 worin
R₁ für Wasserstoff oder Methyl und
r für null oder 1 steht und s, m und n für eine ganze Zahl von 2 bis 6 stehen mit der Maßgabe, daß sofern r für null steht, die Summe aus s und n größer 4 und sofern r für 1 steht, die Summe aus s, m und n größer 6 sein soll,
dadurch gekennzeichnet, daß man ein Dithiol der allgemeinen Formel II
MS-(CH₂)ₛ-[S-(CH₂)ₘ]ᵣ-S-(CH₂)ₙ-SM (II)
worin r, s, m und n die oben bezeichneten Bedeutungen besitzen und M für Wasserstoff oder ein Metallkation steht,
mit mindestens der doppelten molaren Menge eines (Meth)acrylsäurederivats der Formel III 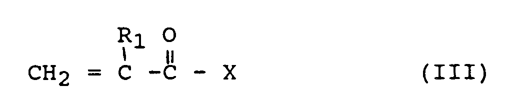 worin
X für 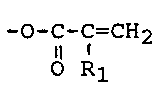 oder für Cl steht und
R₁ Wasserstoff oder Methyl bedeutet,
umsetzt.

5. Verwendung der erfindungsgemäßen Monomeren gemäß den Ansprüchen 1 bis 3 zur Herstellung von hochlichtbrechenden Kunststoffen.

## Claims

1. Dithio(meth)acrylic acid esters of general Formula (I) 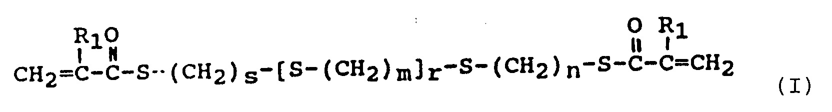 wherein
R₁ is hydrogen or methyl and
r is zero or 1 and s, m and n are integers from 2 to 6, with the proviso that if r is zero, the sum of s and n should be greater than 4, and if r is 1, the sum of s, m and n should he greater than 6.

2. 1,3-Bis(2-methacrylthioethylthio)propane.

3. 1,6-Bis(methacrylthio)-3-thiahexane.

4. A process for preparing Dithio(meth)acrylic acid esters of general Formula I 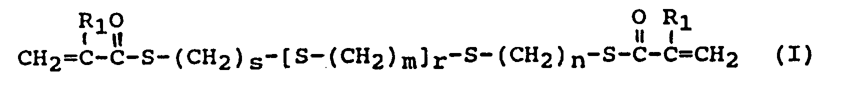 wherein
R₁ is hydrogen or methyl and
r is zero or 1 and s, m and n are integers from 2 to 6 with the proviso that if r is zero the sum of s and n should be greater than 4, and if r is 1, the sum of s, m and n should be greater than 6,
characterised in that a dithiol of general Formula II
MS-(CH₂)ₛ-[S(CH₂)ₘ]ᵣ-S-(CH₂)ₙ-SM (II)
wherein r, s, m and n have the above meanings and M is hydrogen or a metal cation,
is reacted with at least double the molar amount of a (meth)acrylic acid derivative of Formula III 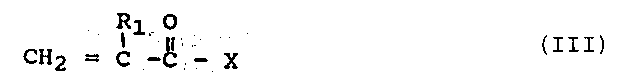 wherein X is 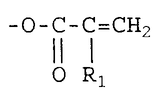 or Cl and
and R₁ is hydrogen or methyl.

5. Use of the monomers according to the invention and as cited in claims 1 to 3 for the preparation of highly refractive plastic materials.

## Revendications

1. Esters d'acide dithio(méth)acrylique de formule générale (I) 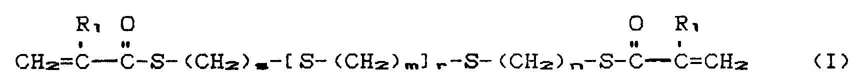 dans laquelle
R₁ est mis pour un atome d'hydrogène ou un reste méthyle,
r est mis pour 0 ou 1 et s, m et n sont mis chacun pour un nombre entier de 2 à 6, étant spécifié qu'au cas où r est mis pour 0, la somme de s et n doit être supérieure à 4 et qu'au cas où r est mis pour 1, la somme de s, m et n doit être supérieure à 6.

2. 1,3-Bis(2-méthacrylthioéthylthio)propane.

3. 1,6-Bis(méthacrylthio)-3-thiahexane.

4. Procédé de préparation d'esters d'acide dithio(méth)acrylique de formule générale (I) 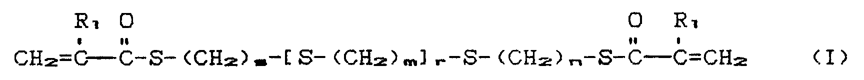 dans laquelle
R₁ est mis pour un atome d'hydrogène ou un reste méthyle,
r est mis pour 0 ou 1 et s, m et n sont mis chacun pour un nombre entier de 2 à 6, étant spécifié qu'au cas où r est mis pour 0, la somme de s et n doit être supérieure à 4 et qu'au cas où r est mis pour 1, la somme de s, m et n doit être supérieure à 6,
caractérisé en ce que l'on fait réagir un dithiol de formule générale (II)
MS-(CH₂)ₛ-[S-(CH₂)ₘ]ᵣ-S-(CH₂)ₙ-SM (II)
dans laquelle
r, s, m et n ont les mêmes significations que précédemment et M est mis pour un atome d'hydrogène ou un cation de métal,
avec la quantité molaire au moins double d'un dérivé d'acide (méth)acrylique de formule (III) 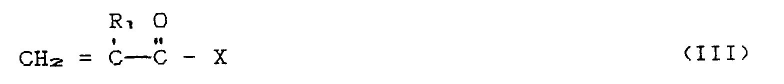 dans laquelle
X est mis pour 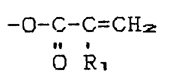 ou pour Cl et
R₁ représente un atome d'hydrogène ou un reste méthyle.

5. Utilisation des monomères conformes à l'invention selon l'une quelconque des revendications 1 à 3 pour la préparation de matières plastiques très réfringentes.
